# EUROPEAN PATENT SPECIFICATION

(11) **EP 4 011 307 B1**
(45) Date of publication and mention of the grant of the patent: **19.02.2025**
(21) Application number: 20850145.2
(22) Date of filing: 31.07.2020
(51) Int. Cl.: A61B 18/12, H03K 3/53, A61N 1/00, A61N 1/32

(54) **REAL-TIME PULSE MONITORING CIRCUIT AND TUMOR TREATMENT APPARATUS**
ECHTZEIT-PULSÜBERWACHUNGSSCHALTUNG UND TUMORBEHANDLUNGSGERÄT
CIRCUIT DE SURVEILLANCE D'IMPULSION EN TEMPS RÉEL ET APPAREIL DE TRAITEMENT DE TUMEUR

(30) Priority: 06.08.2019 CN 201910720830
(43) Date of publication of application: 15.06.2022
(73) Proprietor: Shenzhen Neumann Technology Co., Ltd., Shenzhen, Guangdong 518000 (CN)
(72) Inventor: LAI, Shen, Shenzhen, Guangdong 518000 (CN)
(74) Representative: Marks & Clerk LLP
(86) International application number: PCT/CN2020/106412
(87) International publication number: WO 2021/023129

(56) References cited:
- WO-A1-2006/059067
- WO-A1-2017/151261
- WO-A1-2018/010659
- CN-A- 107 681 916
- CN-A- 107 681 916
- CN-A- 108 702 146
- CN-A- 109 922 862
- CN-A- 110 071 707
- CN-A- 111 374 750
- US-A1- 2018 001 079

## Description

### TECHNICAL FIELD

The present application relates to the field of medical device technique, and particularly to a pulse real-time monitoring circuit and a tumor therapy instrument.

### BACKGROUND

With the development of science and technology, the application of tumor therapy instruments has received wide attention. There are many types of tumor therapy instruments. The working process of the tumor therapy instrument based on an electric pulse technology is generally as follows: the instantaneous electric field intensity of the pulsed electric field applied to the cell is greater than 1kV/cm (kilovolts per centimeter), accordingly, the molecular permeability of the cell is greatly increased, and then the electroporation is produced. As the pulsed electric field intensity continues to increase, irreversible electrical breakdown occurs, which leads to mechanical breakage of the cell membrane until the cell death. The tumor therapy instrument has strict requirements for output pulses. Therefore, the monitoring of the pulses has great significance.

In the implementation process, the inventors found that the conventional technology at least has the following problems: the design of the conventional circuit based on the pulse monitoring of the tumor therapy instrument is complicated, and the real-time performance of the pulse monitoring is unsatisfactory.

The document CN 107681916 A discloses a synergic pulse irreversible electroporation device. The synergic pulse irreversible electroporation device mainly comprises a power supply system, a synergic pulse forming system, a pulse measuring system, a control system and a signal conversion system. According to the synergic pulse irreversible electroporation device, synergic pulse is output to an electrode needle, and the electrode needle is used for carrying out melting on tumor cells. According to the device, the lethal effect of tumor cells can be effectively improved and the melting areas of biological tissues are enlarged by adopting synergic pulse.

The document WO 2017/151261 A1 discloses a sub-microsecond pulsed electric field generator. The field generator includes a controller, which generates a power supply control signal and generates a pulse generator control signal, and a power supply, which receives the power supply control signal and generates one or more power voltages based on the received power supply control signal. The field generator also includes a pulse generator which receives the power voltages and the pulse generator control signal, and generates one or more pulses based on the power voltages and based on the pulse generator control signal. The controller receives feedback signals representing a value of a characteristic of or a result of the pulses and generates at least one of the power supply control signal and the pulse generator control signal based on the received feedback signals.

### SUMMARY

In view of this, as for the problems of complicated design of the conventional circuit based on the pulse monitoring of the tumor therapy instrument and the unsatisfactory real-time performance of the pulse monitoring, it is necessary to provide a pulse real-time monitoring circuit and a tumor therapy instrument.

In order to achieve the above purpose, according to an embodiment of the present invention, a pulse real-time monitoring circuit for a tumor therapy instrument is provided, including a capacitor bank, a pulse generator connected to a positive electrode of the capacitor bank, and a discharge panel connected between the pulse generator and a negative electrode of the capacitor bank; and further including:
a current detection module, a first detection terminal of the current detection module being provided between the pulse generator and the positive electrode of the capacitor bank, and a second detection terminal of the current detection module being provided between the negative electrode of the capacitor bank and the discharge panel, the current detection module being configured to detect a pulse signal outputted by the capacitor bank and obtain a first analog pulse signal;
a voltage detection module, a first detection terminal of the voltage detection module being connected to one end of the discharge panel, and a second detection terminal of the voltage detection module being connected to the other end of the discharge panel; the voltage detection module being configured to detect the pulse signal and obtain a second analog pulse signal;
a controller, the controller being connected to an output terminal of the current detection module and an output terminal of the voltage detection module respectively, the controller being configured to acquire and process the first analog pulse signal and the second analog pulse signal to obtain a pulse current corresponding to the first analog pulse signal and a pulse voltage corresponding to the second analog pulse signal, and transmit the pulse current and pulse voltage to a computer.

In an embodiment, the current detection module includes a first current detection sub-module and a second current detection sub-module;
the first current detection sub-module comprises a first signal conversion module, a second signal conversion module, and a first current sensor provided between the pulse generator and the positive electrode of the capacitor bank;
the second current detection sub-module comprises a third signal conversion module, a fourth signal conversion module, and a second current sensor provided between the negative electrode of the capacitor bank and the discharge panel;
a first input terminal of the first signal conversion module is connected to a first end of the first current sensor, a second input terminal of the first signal conversion module is connected to a second end of the first current sensor, and an output terminal of the first signal conversion module is connected to an input terminal of the second signal conversion module, an output terminal of the second signal conversion module is connected to the controller;
a first input terminal of the third signal conversion module is connected to a first end of the second current sensor, a second input terminal of the third signal conversion module is connected to a second end of the second current sensor, and an output terminal of the third signal conversion module is connected to an input terminal of the fourth signal conversion module, an output terminal of the fourth signal conversion module is connected to the controller.

In an embodiment, the circuit further includes a first primary isolation module connected between the first signal conversion module and the second signal conversion module, a first secondary isolation module connected between the second signal conversion module and the controller, a second primary isolation module connected between the third signal conversion module and the fourth signal conversion module, and a second secondary isolation module connected between the fourth signal conversion module and the controller.

In an embodiment, the first current sensor is an electromagnetic current transformer or an electronic current transformer;
the second current sensor is an electromagnetic current transformer or an electronic current transformer.

In an embodiment, the voltage detection module includes a fifth signal conversion module and a sixth signal conversion module;
a first input terminal of the fifth signal conversion module is connected to one end of the discharge panel, and a second input terminal of the fifth signal conversion module is connected to the other end of the discharge panel, and an output terminal of the fifth signal conversion module is connected to an input terminal of the sixth signal conversion module; an output terminal of the sixth signal conversion module is connected to the controller.

In an embodiment, the circuit further includes a third primary isolation module and a third secondary isolation module;
the third primary isolation module is connected between the output terminal of the fifth signal conversion module and the input terminal of the sixth signal conversion module, and the third secondary isolation module is connected between the output terminal of the sixth signal conversion module and the controller.

In an embodiment, the computer obtains a load impedance according to the received pulse current and the received pulse voltage.

In an embodiment, the controller includes a processing chip and an AD acquisition circuit connected to the processing chip.

In another aspect, according to an embodiment of the present invention, a tumor therapy instrument is provided, including a computer and the pulse real-time monitoring circuit according to any one of the above embodiments;
the computer is connected to the controller.

In an embodiment, the tumor therapy instrument further includes a high-voltage power supply; an output terminal of the high-voltage power supply is connected to a capacitor bank, and a control terminal of the high-voltage power supply is connected to the controller.

A technical solution in the above-mentioned embodiments has the following advantages and beneficial effects.

The pulse generator is connected to the positive electrode of the capacitor bank, the discharge panel is connected between the pulse generator and the negative electrode of the capacitor bank; and a pulse release loop is formed among the pulse generator, the discharge panel and the capacitor bank. The detection terminal of the current detection module (the first detection terminal and the second detection terminal) are connected to both ends of the capacitor bank, the current detection module can detect the pulse signal outputted by the capacitor bank, and then the first analog pulse signal and the second analog pulse signal are obtained. The voltage detection module is connected to both ends of the discharge panel; the voltage detection module can detect the pulse signal outputted by the pulse generator and obtain the third analog pulse signal.

The controller is connected to the current detection module and the voltage detection module respectively; the controller acquires the first analog pulse signal, the second analog pulse signal and the third analog pulse signal, and then respectively transmits the acquired pulse current and pulse voltage to the upper computer, thereby implementing the real-time monitoring of the pulse outputted by the tumor therapy instrument. In the pulse real-time monitoring circuit of each embodiment of the present invention, the circuit structure is simple, and the real-time performance of pulse monitoring is improved.

### BRIEF DESCRIPTION OF THE DRAWINGS

FIG 1 is a schematic structure diagram I of a pulse real-time monitoring circuit according to an embodiment of the present invention.
FIG 2 is a schematic structure diagram II of a pulse real-time monitoring circuit according to an embodiment of the present invention.
FIG 3 is a schematic structure diagram III of a pulse real-time monitoring circuit according to an embodiment of the present invention.
FIG 4 is a schematic block diagram I illustrating a tumor therapy instrument according to an embodiment of the present invention.
FIG 5 is a schematic block diagram II illustrating a tumor therapy instrument according to an embodiment of the present invention.

### DETAILED DESCRIPTION

In order to facilitate the understanding of the present application, the application will be described in a more comprehensive manner with reference to the relevant accompanying drawings. Preferred embodiments of the present invention are shown in the accompanying drawings. However, the present application can be implemented in many different forms and is not limited to the embodiments described herein. Rather, the purpose of providing these embodiments is to make the disclosure of the present application more thorough and comprehensive.

Unless otherwise defined, all technical and scientific terms used herein have the same meaning as commonly understood by those skilled in the technical field of the present application. The terms used in the description of the present application herein are merely for the purpose of describing the specific embodiments, and are not intended to limit the present application. The term "and/or" as used herein includes any and all combinations of one or more related listed items.

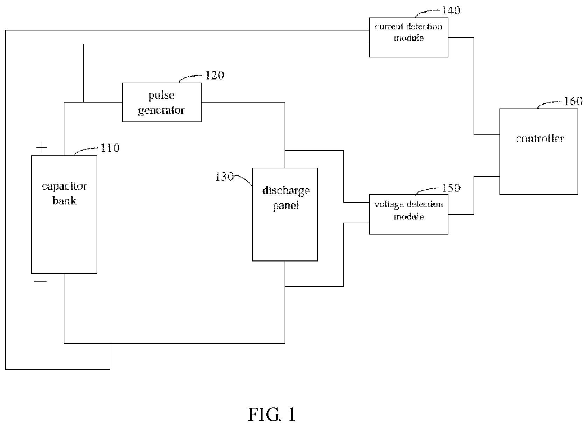

In order to solve the problem of the complicated design of the conventional circuit based on the pulse monitoring of the tumor therapy instrument and the unsatisfactory real-time performance of the pulse monitoring, according to an embodiment, as shown in FIG 1, a pulse real-time monitoring circuit is provided, which includes a capacitor bank 110, a pulse generator 120 connected to a positive electrode of the capacitor bank 110, and a discharge panel 130 connected between the pulse generator 120 and a negative electrode of the capacitor bank 110; and further includes:
a current detection module 140; a first detection terminal of the current detection module 140 is provided between the pulse generator 120 and the positive electrode of the capacitor bank 110, and a second detection terminal of the current detection module 140 is provided between the negative electrode of the capacitor bank 110 and the discharge panel; the current detection module 140 is configured to detect a pulse signal outputted by the capacitor bank 110 and obtain a first analog pulse signal;
a voltage detection module 150; a first detection terminal of the voltage detection module 150 is connected to one end of the discharge panel 130, and a second detection terminal of the voltage detection module 150 is connected to the other end of the discharge panel 130; the voltage detection module 150 is configured to detect the pulse signal and obtain a second analog pulse signal;
a controller 160; the controller 160 is respectively connected to an output terminal of the current detection module 140 and an output terminal of the voltage detection module 150; the controller 160 acquires and processes the first analog pulse signal and the second analog pulse signal to obtain a pulse current corresponding to the first analog pulse signal and a pulse voltage corresponding to the second analog pulse signal, and transmits the pulse current and pulse voltage to an upper computer.

The capacitor bank 110 can be configured to store electrical energy, and can also transmit the stored electrical energy to the pulse generator 120; the capacitor bank 110 can consist of multiple capacitors in parallel and the capacitor bank 110 can also consist of multiple capacitors in series. The pulse generator 120 refers to a system configured to transmit a signal, which is an electrical pulse signal instrument that generates required parameters. For example, the pulse generator 120 can be, but not limited to, a pulse boost pulse generator and a solid-state switch series pulse generator. The discharge panel 130 can be configured to strobe a probe-couple to release pulses; the probe-couple can be configured to output a pulse signal. For example, when the corresponding probe-couple is strobed, a pulse release loop consisting of the capacitor bank 110, the pulse generator 120 and the discharge panel 130 is connected.

The current detection module 140 can be configured to perform a current detection on the pulse signal outputted by the capacitor bank. For example, the current detection module 140 can acquire the pulse signal, and perform conversion (such as filtering and/or attenuation) processing on the pulse signal, to obtain the first analog pulse signal corresponding to the pulse signal. The voltage detection module 150 can be configured to perform a voltage detection on the pulse signal outputted by the pulse generator 120. For example, the voltage detection module 150 can acquire the pulse signal, and perform conversion (such as filtering and/or voltage division) processing on the acquired pulse signal, to obtain the second analog pulse signal corresponding to the pulse signal. The controller 160 refers to a processing device having functions such as signal transmission and signal acquisition and processing. The second analog pulse signal refers to an analog pulse signal obtained after the signal conversion processing; and the pulse voltage refers to a digital pulse voltage. The first analog pulse signal refers to an analog pulse signal obtained after the signal conversion processing; and the pulse current refers to a digital pulse current.

Specifically, the pulse generator 120 is connected to the positive electrode of the capacitor bank 110; and the discharge panel 130 is connected between the pulse generator 120 and the negative electrode of the capacitor bank 110, thereby forming the pulse release loop consisting of the pulse generator 120, the discharge panel 130 and the capacitor bank 110. The detection terminals (the first detection terminal and the second detection terminal) of the current detection module 140 are connected to both ends of the capacitor bank 110, so that the current detection module 140 can detect the pulse signal outputted by the capacitor bank 110, and then obtain the first analog pulse signal. The voltage detection module 150 is connected to both ends of the discharge panel 130; and the voltage detection module 150 can detect the pulse signal outputted by the pulse generator to obtain the second analog pulse signal. The controller 160 is connected to the current detection module 140 and the voltage detection module 150 respectively; and the controller 160 acquires the first analog pulse signal and the second analog pulse signal, and then transmits the acquired pulse current and pulse voltage to the upper computer respectively, to implement the real-time monitoring of the outputted pulse of the tumor therapy instrument.

In the above-mentioned pulse real-time monitoring circuit, the controller acquires the first analog pulse signal and the second analog pulse signal detected by the current detection module, and acquires the third analog pulse signal detected by the voltage detection module, to obtain the corresponding pulse current and pulse voltage, thereby implementing the real-time monitoring of pulse current and pulse voltage, simplifying the structure of pulse monitoring circuit, and improving the real-time performance of pulse monitoring.

In an example, the first analog pulse signal is the analog pulse signal of the first detection terminal; the second analog pulse signal is the analog pulse signal of the second detection terminal; and the control device can process the received analog pulse signal corresponding to the first detection terminal and the analog pulse signal corresponding to the second detection terminal; and the control device can trigger the alarm device timely when a difference value between the analog pulse signal corresponding to the first detection terminal and the analog pulse signal corresponding to the second detection terminal exceeds a leakage safety threshold, thereby implementing a timely response to the leakage alarm of the tumor therapy instrument, and improving the reliability of the leakage response.

In an embodiment, the upper computer obtains a load impedance according to the received pulse current and the received pulse voltage.

The upper computer may be, but not limited to, a tablet computer and a personal computer (PC).

Specifically, the upper computer can process the received pulse current and the received pulse voltage based on a law of resistance to obtain the corresponding load impedance. Thereby, the real-time monitoring of the pulse current, the pulse voltage and the load impedance can be implemented, and the real-time performance of the pulse monitoring is accordingly improved.

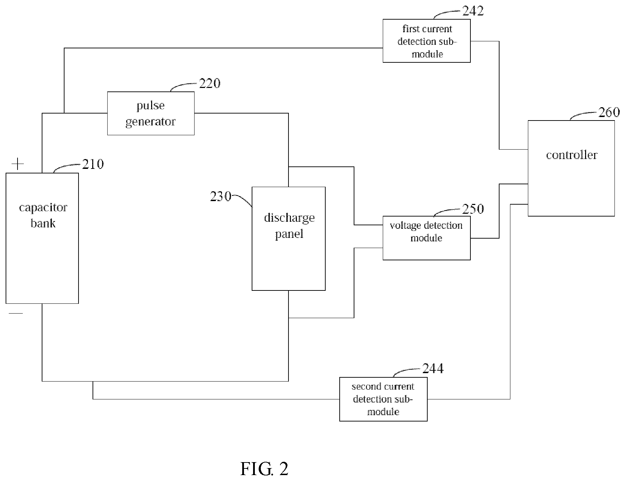

In an embodiment, as shown in FIG 2, a pulse real-time monitoring circuit is provided, which includes a capacitor bank 210, a pulse generator 220 connected to a positive electrode of the capacitor bank 210, and a discharge panel 230 connected between the pulse generator 220 and a negative electrode of the capacitor bank 210; and further includes a current detection module connected to both ends of the capacitor bank 110, a voltage detection module 250 connected to both ends of the discharge panel 230, and a controller 260 connected to the current detection module and the voltage detection module 250 respectively. The current detection module includes a first current detection sub-module 242 and a second current detection sub-module 244. The first current detection sub-module 242 includes a first signal conversion module, a second signal conversion module, and a first current sensor provided between the pulse generator and the positive electrode of the capacitor bank. The second current detection sub-module 244 includes a third signal conversion module, a fourth signal conversion module, and a second current sensor provided between the negative electrode of the capacitor bank and the discharge panel.

A first input terminal of the first signal conversion module is connected to a first end of the first current sensor, a second input terminal of the first signal conversion module is connected to a second end of the first current sensor, and an output terminal of the first signal conversion module is connected to an input terminal of the second signal conversion module; an output terminal of the second signal conversion module is connected to the controller. A first input terminal of the third signal conversion module is connected to a first end of the second current sensor, a second input terminal of the third signal conversion module is connected to a second end of the second current sensor, and an output terminal of the third signal conversion module is connected to an input terminal of the fourth signal conversion module; an output terminal of the fourth signal conversion module is connected to the controller.

The first current sensor refers to a device or module that can sense information of the detected current, and convert the sensed information into an electrical signal satisfying a certain standard according to a certain rule or into information outputs in other required forms. The second current sensor refers to a device or module that can sense information of the detected current, and convert the sensed information into an electrical signal satisfying a certain standard according to a certain rule or into information outputs in other required forms. The first signal conversion module refers to a module capable of scaling the first analog pulse signal; for example, the first signal conversion module can be configured to attenuate the first analog pulse signal of the positive electrode of the capacitor bank, to obtain a converted first analog pulse signal. The second signal conversion module refers to a module which can perform the zooming or scaling conversion on the first analog pulse signal. The third signal conversion module refers to a module that can perform the scaling conversion on the second analog pulse signal; for example, the second signal conversion module can be configured to attenuate the second analog pulse signal of the negative electrode of the capacitor bank, to obtain the converted second analog pulse signal. The fourth signal conversion module refers to a module which can perform the zooming or scaling conversion on the second analog pulse signal.

Specifically, the first current sensor is provided between the pulse generator and the positive electrode of the capacitor bank; the second current sensor is provided between the discharge panel and the negative electrode of the capacitor bank. In the process of outputting the pulse signal by the pulse generator, the first current sensor can acquire the pulse signal corresponding to the positive electrode of the capacitor bank, and process the acquired pulse signal to obtain the first analog pulse signal corresponding to the positive electrode of the capacitor bank. The second current sensor can acquire the pulse signal corresponding to the negative electrode of the capacitor bank, and process the acquired pulse signal to obtain the first analog pulse signal corresponding to the negative electrode of the capacitor bank. The first current sensor can transmit the first analog pulse signal corresponding to the positive electrode of the capacitor bank to the first signal conversion module; and the first signal conversion module can perform primary conversion processing on the received first analog pulse signal, and transmit the first analog pulse signal obtained after the primary conversion processing to the second signal conversion module; and then the second signal conversion module can perform secondary conversion processing on the received first analog pulse signal after the primary conversion processing to obtain the first analog pulse signal after the secondary conversion processing; so that the controller can acquire the first analog pulse signal after the two-stage processing, and transmit the acquired pulse current to the upper computer.

The second current sensor can transmit the second analog pulse signal corresponding to the negative electrode of the capacitor bank to the third signal conversion module, and the third signal conversion module can perform the primary conversion processing on the received second analog pulse signal, and transmit the second analog pulse signal obtained after the primary conversion processing to the fourth signal conversion module; and the fourth signal conversion module can perform secondary conversion processing on the received second analog pulse signal after the primary conversion processing, to obtain the second analog pulse signal after the secondary conversion processing; so that the controller can acquire the second analog pulse signal after the two-stage processing, and then transmit the acquired pulse current to the upper computer, to implement the real-time monitoring of the pulse current of the tumor therapy instrument.

It should be noted that the first analog pulse signal is an analog pulse voltage signal; the second analog pulse signal is an analog pulse voltage signal. The controller can acquire the analog pulse voltage signal to obtain a corresponding digital pulse voltage signal, and perform voltage-current conversion processing on the digital pulse voltage signal to obtain the pulse current.

In the above-mentioned pulse real-time monitoring circuit, the first signal conversion module performs the primary conversion processing on the first analog pulse signal transmitted by the first current sensor, and the second signal conversion module performs the secondary conversion processing on the first analog pulse signal after the primary conversion processing transmitted by the first signal conversion module, so that the first analog pulse signal after the two-stage conversion processing can meet the requirements of the parameter acquisition of the controller. The third signal conversion module performs the primary conversion processing on the second analog pulse signal transmitted by the second current sensor, and the fourth signal conversion module performs the secondary conversion processing on the second analog pulse signal after the primary conversion processing transmitted by the third signal conversion module, so that the second analog pulse signal after the two-stage conversion processing can meet the requirements of the parameter acquisition of the controller, and meanwhile the accuracy of the pulse current monitoring is improved.

In an embodiment, as shown in FIG 2, the pulse real-time monitoring circuit further includes a first primary isolation module connected between the first signal conversion module and the second signal conversion module, a first secondary isolation module connected between the second signal conversion module and the controller, a second primary isolation module connected between the third signal conversion module and the fourth signal conversion module, and a second secondary isolation module connected between the fourth signal conversion module and the controller.

The first primary isolation module may be, but not limited to, an external optical circuit photocoupler and an internal optical circuit photocoupler; the first secondary isolation module may be, but not limited to, an external optical circuit photocoupler and an internal optical circuit photocoupler. The second primary isolation module can be, but is not limited to, an external optical circuit photocoupler and an internal optical circuit photocoupler; the second secondary isolation module can be, but is not limited to, an external optical circuit photocoupler and an internal optical circuit photocoupler.

Specifically, the first primary isolation module is connected between the first signal conversion module and the second signal conversion module; the first signal conversion module transmits the converted signal to the first primary isolation module to perform the isolation conversion processing on the pulse signal by the first primary isolation module, and transmits the processed pulse signal to the second signal conversion module, so that the second signal conversion module performs the conversion processing on the pulse signal, and transmits the first analog pulse signal obtained after the conversion processing to the first secondary isolation module, thereby isolating the pulse signal from the first analog pulse signal obtained after the primary conversion processing, and improving the anti-interference capability of the signal transmission on the pulse generator side. The first secondary isolation module is connected between the second signal conversion module and the controller. The first secondary isolation module receives the converted first analog pulse signal transmitted by the second signal conversion module, and performs the isolation conversion processing on the first analog pulse signal obtained after the secondary conversion processing, and transmits the processed first analog pulse signal to the controller, so that the controller obtains the pulse current according to the first analog pulse signal, thereby isolating the first analog pulse signal after the secondary conversion processing from the pulse current, and improving the anti-interference capability of the signal transmission on the controller side.

The second primary isolation module is connected between the third signal conversion module and the fourth signal conversion module; and the third signal conversion module transmits the converted signal to the second primary isolation module to perform the isolation conversion processing on the pulse signal by the second primary isolation module, and transmits the processed pulse signal to the fourth signal conversion module, so that the fourth signal conversion module performs the conversion processing on the pulse signal, and transmits the second analog pulse signal obtained after the conversion processing to the second secondary isolation module, thereby isolating the pulse signal from the second analog pulse signal after the primary conversion processing, and improving the anti-interference capability of the signal transmission on the pulse generator side. The second secondary isolation module is connected between the fourth signal conversion module and the controller. The second secondary isolation module receives the converted second analog pulse signal transmitted by the fourth signal conversion module and performs the isolation conversion processing on the second analog pulse signal obtained after the secondary conversion processing, and transmits the processed second analog pulse signal to the controller, so that the controller obtains the pulse current according to the second analog pulse signal, thereby isolating the second analog pulse signal obtained after the secondary conversion processing from the pulse current, and improving the anti-interference capability of the signal transmission on the controller side.

It should be noted that the number of the first primary isolation modules is at least one. For example, when the pulse real-time monitoring circuit includes two or more first primary isolation modules, each of the first primary isolation modules is connected in series between the first signal conversion module and the second signal conversion module. The number of the first secondary isolation modules is at least one. For example, when the pulse real-time monitoring circuit includes two or more first secondary isolation modules, each of the first secondary isolation modules is connected in series between the second signal conversion module and the controller. The number of the second primary isolation modules is at least one. For example, when the pulse real-time monitoring circuit includes two or more second primary isolation modules, each of the second primary isolation modules is connected in series between the third signal conversion module and the fourth signal conversion module. The number of the second secondary isolation modules is at least one. For example, when the pulse real-time monitoring circuit includes two or more second secondary isolation modules, each of the second secondary isolation modules is connected in series between the fourth signal conversion module and the controller.

In an example, the first primary isolation module can be a linear optocoupler isolator; the first secondary isolation module can be a linear optocoupler isolator. The second primary isolation module can be a linear optocoupler isolator; the second secondary isolation module can be a linear optocoupler isolator.

In a specific embodiment, the first current sensor is an electromagnetic current transformer or an electronic current transformer. The second current sensor is an electromagnetic current transformer or an electronic current transformer.

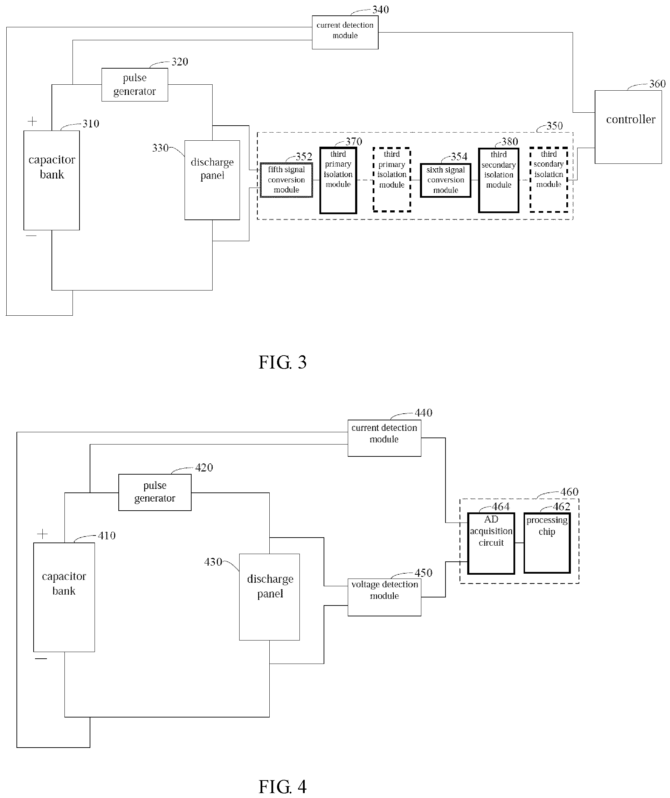

In an embodiment, as shown in FIG 3, a pulse real-time monitoring circuit is provided, which includes a capacitor bank 310, a pulse generator 320 connected to a positive electrode of the capacitor bank 310, and a discharge panel 330 connected between the pulse generator 320 and a negative electrode of the capacitor bank 310; and further includes a current detection module 340 connected to both ends of the capacitor bank 310, a voltage detection module 350 connected to both ends of the discharge panel 330, and a controller 360 connected to the current detection module 340 and the voltage detection module 350 respectively. The voltage detection module 350 includes a fifth signal conversion module 352 and a sixth signal conversion module 354.

A first input terminal of the fifth signal conversion module 352 is connected to one end of the discharge panel 330, and a second input terminal of the fifth signal conversion module 352 is connected to the other end of the discharge panel 330, and an output terminal of the fifth signal conversion module 352 is connected to an input terminal of the sixth signal conversion module 354; an output terminal of the sixth signal conversion module 354 is connected to the controller 360.

Specifically, the fifth signal conversion module 352 may include a first detection terminal, a second detection terminal and an output terminal. The first detection terminal of the fifth signal conversion module 352 is connected to one end of the discharge panel 330; the second detection terminal of the voltage detection circuit 352 is connected to the other end of the discharge panel 330; and the output terminal of the voltage detection circuit 352 is connected to the sixth signal conversion module 354; the output terminal of the sixth signal conversion module 354 is connected to the controller 360. The pulse signal outputted by the pulse generator 320 can be scaled by the fifth signal conversion module 352 and the sixth signal conversion module 354 in sequence, and then a third analog pulse signal after the two-stage conversion processing can be obtained, so that the controller 360 can acquire the processed third analog pulse signal and transmit the acquired pulse voltage to the upper computer, thereby implementing the real-time monitoring of the pulse voltage of the tumor therapy instrument.

In the above-mentioned pulse real-time monitoring circuit, the fifth signal conversion module and the sixth signal conversion module sequentially perform the proportional scaling conversion on the pulse signal, so that the converted third analog pulse signal can meet the requirements of the parameter acquisition of the controller, and meanwhile the accuracy of the monitoring of the pulse voltage is improved.

In an embodiment, as shown in FIG 3, the circuit further includes a third primary isolation module 370 and a third secondary isolation module 380. The third primary isolation module 370 is connected between the output terminal of the fifth signal conversion module 352 and the input terminal of the sixth signal conversion module 354; the third secondary isolation module 380 is connected between the output terminal of the sixth signal conversion module 354 and the controller 360.

The third primary isolation module 370 may be, but not limited to, an external optical circuit photocoupler and an internal optical circuit photocoupler; the third secondary isolation module 380 may be, but not limited to, an external optical circuit photocoupler and an internal optical circuit photocoupler.

Specifically, the third primary isolation module 370 is connected between the fifth signal conversion module 352 and the sixth signal conversion module 354; the pulse generator 320 can transmit the outputted pulse signal to the third primary isolation module 370 to perform the isolation conversion processing on the pulse signal by the third primary isolation module 370, and transmit the second analog pulse signal after the primary conversion processing to the sixth signal conversion module 354, thereby isolating the third analog pulse signal after the primary conversion processing from the third analog pulse signal after the secondary conversion processing, and further improving the anti-interference capability of the signal transmission on the pulse generator side. The third secondary isolation module 380 is connected between the sixth signal conversion module 354 and the controller 360, such that the sixth signal conversion module 354 performs the isolation conversion processing on the received signal, and transmits the third analog pulse signal obtained after the secondary conversion processing to the controller 360, so that the controller 360 obtains the pulse voltage according to the third analog pulse signal, thereby isolating the third analog pulse signal after the secondary conversion processing from the pulse voltage, and further improving the anti-interference capability of the signal transmission on the controller side.

It should be noted that the number of the third primary isolation modules is at least one. For example, when the pulse real-time monitoring circuit includes two or more third primary isolation modules, each of the third primary isolation modules is connected in series between the output terminal of the fifth signal conversion module and the input terminal of the sixth signal conversion module. The number of the third secondary isolation modules is at least one. For example, when the pulse real-time monitoring circuit includes two or more third secondary isolation modules, each of the third secondary isolation modules is connected in series between the output terminal of the sixth signal conversion module and the controller.

In an example, the third primary isolation module 370 can be a linear optocoupler isolator; the third secondary isolation module 380 can be a linear optocoupler isolator.

In an embodiment, as shown in FIG 4, a pulse real-time monitoring circuit is provided, which includes a capacitor bank 410, a pulse generator 420 connected to a positive electrode of the capacitor bank 410, and a discharge panel 430 connected between the pulse generator 420 and a negative electrode of the capacitor bank 410; and further includes a current detection module 440 connected to both ends of the capacitor bank 410, a voltage detection module 450 connected to both ends of the discharge panel 430, and a controller 460 connected to the current detection module 440 and the voltage detection module 450 respectively. The controller 460 includes a processing chip 462 and an AD acquisition circuit 464 connected to the processing chip 462.

The processing chip 462 may be, but not limited to, a single-chip microcomputer chip, an ARM processing chip, and an FPGA processing chip. The AD acquisition circuit 464 refers to an acquisition circuit capable of performing an analog-to-digital conversion on the signal.

Specifically, the processing chip 462 is connected to the AD acquisition circuit 464, and the AD acquisition circuit 464 may include a first acquisition port and a second acquisition port. The first acquisition port is connected to the current detection module 440; the second acquisition port is connected to the voltage detection module 450. The processing chip 462 can drive the AD acquisition circuit 464 to operate, and the AD acquisition circuit 464 can acquire the first analog pulse signal and then the second analog pulse signal through the first acquisition port, and convert the first analog pulse signal and the second analog pulse signal into the digital pulse current through the analog-to-digital conversion processing, and transmit the digital pulse current to the processing chip 462; the processing chip 462 can transmit the digital pulse current to the upper computer, and the upper computer monitors the pulse current in real time.

Similarly, the processing chip 462 can drive the AD acquisition circuit 464 to operate, and then the AD acquisition circuit 464 can acquire the third analog pulse signal through the second acquisition port, and convert the third analog pulse signal into the digital pulse voltage through the analog-to-digital conversion processing, and transmit the digital pulse voltage to the processing chip 462; the processing chip 462 can transmit the digital pulse voltage to the upper computer, and the upper computer monitors the pulse voltage in real time. In the above-mentioned pulse real-time monitoring circuit, the pulse monitoring circuit has a simple structure, which improves the real-time performance of the pulse monitoring.

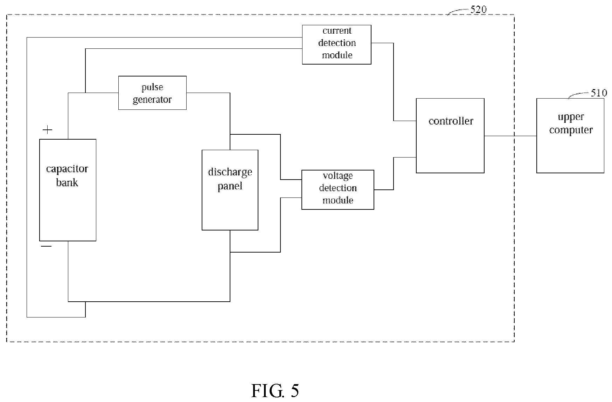

In an embodiment, as shown in FIG 5, a tumor therapy instrument is provided, which includes an upper computer 510 and a pulse real-time monitoring circuit 520 in any one of the above-mentioned embodiments; the upper computer 510 is connected to a controller.

The upper computer 510 may be, but not limited to, a tablet computer and a personal computer (PC).

Specifically, the pulse real-time monitoring circuit 520 may include a capacitor bank, a pulse generator connected to the positive electrode of the capacitor bank, and a discharge panel connected between the pulse generator and the negative electrode of the capacitor group; and may further include a current detection module connected to both ends of the capacitor bank, a voltage detection module connected to both ends of the discharge board, and a controller connected to the current detection module and the voltage detection module respectively. The current detection module can detect the pulse signals of the positive and negative electrodes of the capacitor bank, and then obtain the first analog pulse signal and the second analog pulse signal; the controller acquires the first analog pulse signal and the second analog pulse signal, and then can transmit the acquired pulse current to the upper computer to implement the real-time monitoring of the pulse current of the pulse outputted by the tumor therapy instrument. The voltage detection module can detect the pulse signal outputted by the pulse generator, and obtain the third analog pulse signal, and then obtain the third analog pulse signal; the controller acquires the third analog pulse signal, and then transmits the acquired pulse voltage to the upper computer, to implement the real-time monitoring of the pulse voltage of the pulse outputted by the tumor therapy instrument. The circuit structure of the tumor therapy instrument is simple, and the real-time performance of the pulse monitoring is improved.

Further, the upper computer 510 can also process the acquired pulse current and pulse voltage based on a law of resistance, and then obtain the corresponding load impedance, thereby implementing the real-time monitoring of the pulse current, pulse voltage and the load impedance. The upper computer can also display a waveform diagram corresponding to the pulse current, the pulse voltage and the load impedance, so that the user can observe the corresponding waveform diagram and control the tumor therapy instrument according to the waveform diagram.

In an embodiment, the tumor therapy instrument further includes a high-voltage power supply; an output terminal of the high-voltage power supply is connected to the capacitor bank, and a control terminal of the high-voltage power supply is connected to the controller.

The high-voltage power supply may be a DC high-voltage power supply, and an output power supply of the high-voltage power supply may be a kilovolt-level power supply.

The high-voltage power supply is connected between the controller and the capacitor bank; the controller may control the high-voltage power supply, so that the high-voltage power supply charges the capacitor bank.

The technical features of the above-mentioned embodiments can be combined arbitrarily. In order to make the description concise, all possible combinations of the various technical features in the above-mentioned embodiments are not described. However, as long as there is no contradiction in the combination of these technical features, all should be considered as the scope of the present application.

The above-mentioned embodiments are merely some embodiments of the present invention, and their descriptions are more specific and detailed, but they should not be understood as limiting the scope of the present application. It should be pointed out that those of ordinary skill in the art can make several modifications and improvements without departing from the concept of the present application, and these all fall within the protection scope of the present application. Therefore, the scope of protection of the present application shall be subject to the appended claims.

## Claims

1. A pulse real-time monitoring circuit for a tumor therapy instrument, comprising a capacitor bank (110), a pulse generator (120) connected to a positive electrode of the capacitor bank (110), and a discharge panel (130) connected between the pulse generator (120) and a negative electrode of the capacitor bank (110); and further comprising:
a current detection module (140), wherein a first detection terminal of the current detection module (140) is provided between the pulse generator (120) and the positive electrode of the capacitor bank (110), and a second detection terminal of the current detection module (140) is provided between the negative electrode of the capacitor bank (110) and the discharge panel (130), the current detection module (140) is configured to detect a pulse signal outputted by the capacitor bank (110) and obtain a first analog pulse signal;
a voltage detection module (150), wherein a first detection terminal of the voltage detection module (150) is connected to one end of the discharge panel (130), and a second detection terminal of the voltage detection module (150) is connected to the other end of the discharge panel (130); the voltage detection module (150) is configured to detect the pulse signal and obtain a second analog pulse signal;
a controller (160), wherein the controller (160) is connected to an output terminal of the current detection module (140) and an output terminal of the voltage detection module (150) respectively, the controller (160) is configured to acquire and process the first analog pulse signal and the second analog pulse signal to obtain a pulse current corresponding to the first analog pulse signal and a pulse voltage corresponding to the second analog pulse signal, and transmit the pulse current and pulse voltage to a computer.

2. The pulse real-time monitoring circuit for a tumor therapy instrument according to claim 1, wherein the current detection module (140) comprises a first current detection sub-module (242) and a second current detection sub-module (244);
the first current detection sub-module (242) comprises a first signal conversion module, a second signal conversion module, and a first current sensor provided between the pulse generator and the positive electrode of the capacitor bank;
the second current detection sub-module (244) comprises a third signal conversion module, a fourth signal conversion module, and a second current sensor provided between the negative electrode of the capacitor bank and the discharge panel;
a first input terminal of the first signal conversion module is connected to a first end of the first current sensor, a second input terminal of the first signal conversion module is connected to a second end of the first current sensor, and an output terminal of the first signal conversion module is connected to an input terminal of the second signal conversion module, an output terminal of the second signal conversion module is connected to the controller (160);
a first input terminal of the third signal conversion module is connected to a first end of the second current sensor, a second input terminal of the third signal conversion module is connected to a second end of the second current sensor, and an output terminal of the third signal conversion module is connected to an input terminal of the fourth signal conversion module, an output terminal of the fourth signal conversion module is connected to the controller (160).

3. The pulse real-time monitoring circuit for a tumor therapy instrument according to claim 2, further comprising a first primary isolation module connected between the first signal conversion module and the second signal conversion module, a first secondary isolation module connected between the second signal conversion module and the controller (160), a second primary isolation module connected between the third signal conversion module and the fourth signal conversion module, and a second secondary isolation module connected between the fourth signal conversion module and the controller (160).

4. The pulse real-time monitoring circuit for a tumor therapy instrument according to claim 3, wherein the first current sensor is an electromagnetic current transformer or an electronic current transformer;
the second current sensor is an electromagnetic current transformer or an electronic current transformer.

5. The pulse real-time monitoring circuit for a tumor therapy instrument according to claim 1, wherein the voltage detection module (150) comprises a fifth signal conversion module (352) and a sixth signal conversion module (354);
a first input terminal of the fifth signal conversion module (352) is connected to one end of the discharge panel (130), and a second input terminal of the fifth signal conversion module (352) is connected to the other end of the discharge panel (130), and an output terminal of the fifth signal conversion module (352) is connected to an input terminal of the sixth signal conversion module (354); an output terminal of the sixth signal conversion module (354) is connected to the controller (160).

6. The pulse real-time monitoring circuit for a tumor therapy instrument according to claim 5, further comprising a third primary isolation module (370) and a third secondary isolation module (380);
wherein the third primary isolation module (370) is connected between the output terminal of the fifth signal conversion module (352) and the input terminal of the sixth signal conversion module (354), and the third secondary isolation module (380) is connected between the output terminal of the sixth signal conversion module (354) and the controller (160).

7. The pulse real-time monitoring circuit for a tumor therapy instrument according to claim 1, wherein the computer obtains a load impedance according to the received pulse current and the received pulse voltage.

8. The pulse real-time monitoring circuit for a tumor therapy instrument according to any one of claims 1 to 7, wherein the controller (160) comprises a processing chip (462) and an AD acquisition circuit (464) connected to the processing chip (462).

9. A tumor therapy instrument, comprising a computer (510), and the pulse real-time monitoring circuit (520) according to any one of claims 1 to 8;
wherein the computer (510) is connected to the controller (160).

10. The tumor therapy instrument according to claim 9, further comprising a high-voltage power supply; wherein an output terminal of the high-voltage power supply is connected to the capacitor bank (110), and a control terminal of the high-voltage power supply is connected to the controller (160).

## Patentansprüche

1. Puls-Echtzeitüberwachungsschaltung für ein Tumortherapieinstrument, die eine Kondensatorbank (110), einen Pulsgenerator (120), der mit einer positiven Elektrode der Kondensatorbank (110) verbunden ist, und eine Entladungsplatte (130), die zwischen dem Pulsgenerator (120) und einer negativen Elektrode der Kondensatorbank (110) angeschlossen ist, umfasst; und ferner Folgendes umfasst:
ein Stromermittlungsmodul (140), wobei ein erster Ermittlungsanschluss des Stromermittlungsmoduls (140) zwischen dem Pulsgenerator (120) und der positiven Elektrode der Kondensatorbank (110) bereitgestellt wird, und ein zweiter Ermittlungsanschluss des Stromermittlungsmoduls (140) zwischen der negativen Elektrode der Kondensatorbank (110) und der Entladungsplatte (130) bereitgestellt wird, wobei das Stromermittlungsmodul (140) dafür konfiguriert ist, ein Pulssignal zu ermitteln, das durch die Kondensatorbank (110) ausgegeben wird, und ein erstes analoges Pulssignal zu gewinnen,
ein Spannungsermittlungsmodul (150), wobei ein erster Ermittlungsanschluss des Spannungsermittlungsmoduls (150) mit einem Ende der Entladungsplatte (130) verbunden ist, und ein zweiter Ermittlungsanschluss des Spannungsermittlungsmoduls (150) mit dem anderen Ende der Entladungsplatte (130) verbunden ist; das Spannungsermittlungsmodul (150) dafür konfiguriert ist, das Pulssignal zu ermitteln und ein zweites analoges Pulssignal zu gewinnen,
eine Steuerung (160), wobei die Steuerung (160) mit einem Ausgangsanschluss des Stromermittlungsmoduls (140) beziehungsweise einem Ausgangsanschluss des Spannungsermittlungsmoduls (150) verbunden ist, wobei die Steuerung (160) dafür konfiguriert ist, das erste analoge Pulssignal und das zweite analoge Pulssignal zu erfassen und zu verarbeiten, um einen Pulsstrom, der dem ersten analogen Pulssignal entspricht, und eine Pulsspannung, die dem zweiten analogen Pulssignal entspricht, zu gewinnen, und den Pulsstrom und die Pulsspannung an einen Rechner zu übermitteln.

2. Puls-Echtzeitüberwachungsschaltung für ein Tumortherapieinstrument nach Anspruch 1, wobei das Stromermittlungsmodul (140) ein erstes Stromermittlungsuntermodul (242) und ein zweites Stromermittlungsuntermodul (244) umfasst,
das erste Stromermittlungsuntermodul (242) ein erstes Signalumwandungsmodul, ein zweites Signalumwandungsmodul und einen ersten Stromsensor, der zwischen dem Pulsgenerator und der positiven Elektrode der Kondensatorbank bereitgestellt ist, umfasst,
das zweite Stromermittlungsuntermodul (244) ein drittes Signalumwandungsmodul, ein viertes Signalumwandungsmodul und einen zweiten Stromsensor, der zwischen der negativen Elektrode der Kondensatorbank und der Entladungsplatte bereitgestellt ist, umfasst,
ein erster Eingangsanschluss des ersten Signalumwandungsmoduls mit einem ersten Ende des ersten Stromsensors verbunden ist, ein zweiter Eingangsanschluss des ersten Signalumwandungsmoduls mit einem zweiten Ende des ersten Stromsensors verbunden ist, und ein Ausgangsanschluss des ersten Signalumwandungsmoduls mit einem Eingangsanschluss des zweiten Signalumwandungsmoduls verbunden ist, ein Ausgangsanschluss des zweiten Signalumwandungsmoduls mit der Steuerung (160) verbunden ist,
ein erster Eingangsanschluss des dritten Signalumwandungsmoduls mit einem ersten Ende des zweiten Stromsensors verbunden ist, ein zweiter Eingangsanschluss des dritten Signalumwandungsmoduls mit einem zweiten Ende des zweiten Stromsensors verbunden ist, und ein Ausgangsanschluss des dritten Signalumwandungsmoduls mit einem Eingangsanschluss des vierten Signalumwandungsmoduls verbunden ist, ein Ausgangsanschluss des vierten Signalumwandungsmoduls mit der Steuerung (160) verbunden ist.

3. Puls-Echtzeitüberwachungsschaltung für ein Tumortherapieinstrument nach Anspruch 2, die ferner ein erstes primäres Isolationsmodul, das zwischen dem ersten Signalumwandlungsmodul und dem zweiten Signalumwandlungsmodul angeschlossen ist, ein erstes sekundäres Isolationsmodul, das zwischen dem zweiten Signalumwandlungsmodul und der Steuerung (160) angeschlossen ist, ein zweites primäres Isolationsmodul, das zwischen dem dritten Signalumwandlungsmodul und dem vierten Signalumwandlungsmodul angeschlossen ist, und ein zweites sekundäres Isolationsmodul, das zwischen dem vierten Signalumwandlungsmodul und der Steuerung (160) angeschlossen ist, umfasst.

4. Puls-Echtzeitüberwachungsschaltung für ein Tumortherapieinstrument nach Anspruch 3, wobei der erste Stromsensor ein elektromagnetischer Stromwandler oder ein elektronischer Stromwandler ist,
der zweite Stromsensor ein elektromagnetischer Stromwandler oder ein elektronischer Stromwandler ist.

5. Puls-Echtzeitüberwachungsschaltung für ein Tumortherapieinstrument nach Anspruch 1, wobei das Spannungsermittlungsmodul (150) ein fünftes Signalumwandlungsmodul (352) und ein sechstes Signalumwandlungsmodul (354) umfasst,
ein erster Eingangsanschluss des fünften Signalumwandlungsmoduls (352) mit einem Ende der Entladungsplatte (130) verbunden ist, und ein zweiter Eingangsanschluss des fünften Signalumwandlungsmoduls (352) mit dem anderen Ende der Entladungsplatte (130) verbunden ist, und ein Ausgangsanschluss des fünften Signalumwandlungsmodule (352) mit einem Eingangsanschluss des sechsten Signalumwandlungsmoduls (354) verbunden ist; ein Ausgangsanschluss des sechsten Signalumwandlungsmoduls (354) mit der Steuerung (160) verbunden ist.

6. Puls-Echtzeitüberwachungsschaltung für ein Tumortherapieinstrument nach Anspruch 5, die ferner ein drittes primäres Isolationsmodul (370) und ein drittes sekundäres Isolationsmodul (380) umfasst,
wobei das dritte primäre Isolationsmodul (370) zwischen dem Ausgangsanschluss des fünften Signalumwandungsmoduls (352) und dem Eingangsanschluss des sechsten Signalumwandlungsmoduls (354) angeschlossen ist, und das dritte sekundäre Isolationsmodul (380) zwischen dem Ausgangsanschluss des sechsten Signalumwandlungsmoduls (354) und der Steuerung (160) angeschlossen ist.

7. Puls-Echtzeitüberwachungsschaltung für ein Tumortherapieinstrument nach Anspruch 1, wobei der Rechner eine Lastimpedanz gemäß dem empfangenen Pulsstrom und der empfangenen Pulsspannung gewinnt.

8. Puls-Echtzeitüberwachungsschaltung für ein Tumortherapieinstrument nach einem der Ansprüche 1 bis 7, wobei die Steuerung (160) einen Verarbeitungschip (462) und eine AD-Erfassungsschaltung (464), die mit dem Verarbeitungschip (462) verbunden ist, umfasst.

9. Tumortherapieinstrument, das einen Rechner (510) und die Puls-Echtzeitüberwachungsschaltung (520) nach einem der Ansprüche 1 bis 8 umfasst,
wobei der Rechner (510) mit der Steuerung (160) verbunden ist.

10. Tumortherapieinstrument nach Anspruch 9, das ferner eine Hochspannungsenergieversorgung umfasst; wobei ein Ausgangsanschluss der Hochspannungsenergieversorgung mit der Kondensatorbank (110) verbunden ist, und ein Steueranschluss der Hochspannungsenergieversorgung mit der Steuerung (160) verbunden ist.

## Revendications

1. Circuit de surveillance en temps réel d'impulsions pour un instrument de thérapie d'une tumeur, comprenant un banc de condensateurs (110), un générateur d'impulsions (120) raccordé à une électrode positive du banc de condensateurs (110), et un panneau de décharge (130) connecté entre le générateur d'impulsions (120) et une électrode négative du banc de condensateurs (110) ; et comprenant en outre :
un module de détection de courant (140), dans lequel un premier terminal de détection du module de détection de courant (140) est fourni entre le générateur d'impulsions (120) et l'électrode positive du banc de condensateurs (110), et un deuxième terminal de détection du module de détection de courant (140) est fourni entre l'électrode négative du banc de condensateurs (110) et le panneau de décharge (130), le module de détection de courant (140) est configuré pour détecter un signal d'impulsion émis en sortie par le banc de condensateurs (110) et obtenir un premier signal d'impulsion analogique ;
un module de détection de tension (150), dans lequel un premier terminal de détection du module de détection de tension (150) est connecté à une extrémité du panneau de décharge (130), et un deuxième terminal de détection du module de détection de tension (150) est connecté à l'autre extrémité du panneau de décharge (130) ; le module de détection de tension (150) est configuré pour détecter le signal d'impulsion et obtenir un deuxième signal d'impulsion analogique ;
un dispositif de commande (160), dans lequel le dispositif de commande (160) est raccordé à un terminal de sortie du module de détection de courant (140) et un terminal de sortie du module de détection de tension (150) respectivement, le dispositif de commande (160) est configuré pour acquérir et traiter le premier signal d'impulsion analogique et le deuxième signal d'impulsion analogique pour obtenir un courant d'impulsion correspondant au premier signal d'impulsion analogique et une tension d'impulsion correspondant au deuxième signal d'impulsion analogique, et transmettre le courant d'impulsion et la tension d'impulsion à un ordinateur.

2. Circuit de surveillance en temps réel d'impulsions pour un instrument de thérapie d'une tumeur selon la revendication 1, dans lequel le module de détection de courant (140) comprend un premier sous-module de détection de courant (242) et un deuxième sous-module de détection de courant (244) ;
le premier sous-module de détection de courant (242) comprend un premier module de conversion de signal, un deuxième module de conversion de signal et un premier capteur de courant fourni entre le générateur d'impulsion et l'électrode positive du banc de condensateurs ;
le deuxième sous-module de détection de courant (244) comprend un troisième module de conversion de signal, un quatrième module de conversion de signal et un deuxième capteur de courant fourni entre l'électrode négative du banc de condensateurs et le panneau de décharge ;
un premier terminal d'entrée du premier module de conversion de signal est connecté à une première extrémité du premier capteur de courant, un deuxième terminal d'entrée du premier module de conversion de signal est connecté à une deuxième extrémité du premier capteur de courant, et un terminal de sortie du premier module de conversion de signal est connecté à un terminal d'entrée du deuxième module de conversion de signal, un terminal de sortie du deuxième module de conversion de signal est connecté au dispositif de commande (160) ;
un premier terminal d'entrée du troisième module de conversion de signal est connecté à une première extrémité du deuxième capteur de courant, un deuxième terminal d'entrée du troisième module de conversion de signal est connecté à une deuxième extrémité du deuxième capteur de courant, et un terminal de sortie du troisième module de conversion de signal est connecté à un terminal d'entrée du quatrième module de conversion de signal, un terminal de sortie du quatrième module de conversion de signal est connecté au dispositif de commande (160).

3. Circuit de surveillance en temps réel d'impulsions pour un instrument de thérapie d'une tumeur selon la revendication 2, comprenant en outre un premier module d'isolation primaire connecté entre le premier module de conversion de signal et le deuxième module de conversion de signal, un premier module d'isolation secondaire connecté entre le deuxième module de conversion de signal et le dispositif de commande (160), un deuxième module d'isolation primaire connecté entre le troisième module de conversion de signal et le quatrième module de conversion de signal, et un deuxième module d'isolation secondaire connecté entre le quatrième module de conversion de signal et le dispositif de commande (160).

4. Circuit de surveillance en temps réel d'impulsions pour un instrument de thérapie d'une tumeur selon la revendication 3, dans lequel le premier capteur de courant est un transformateur de courant électromagnétique ou un transformateur de courant électronique ;
le deuxième capteur de courant est un transformateur de courant électromagnétique ou un transformateur de courant électronique.

5. Circuit de surveillance en temps réel d'impulsions pour un instrument de thérapie d'une tumeur selon la revendication 1, dans lequel le module de détection de tension (150) comprend un cinquième module de conversion de signal (352) et un sixième module de conversion de signal (354) ;
un premier terminal d'entrée du cinquième module de conversion de signal (352) est connecté à une extrémité du panneau de décharge (130), et un deuxième terminal d'entrée du cinquième module de conversion de signal (352) est connecté à l'autre extrémité du panneau de décharge (130), et un terminal de sortie du cinquième module de conversion de signal (352) est connecté à un terminal d'entrée du sixième module de conversion de signal (354) ; un terminal de sortie du sixième module de conversion de signal (354) est connecté au dispositif de commande (160).

6. Circuit de surveillance en temps réel d'impulsions pour un instrument de thérapie d'une tumeur selon la revendication 5, comprenant en outre un troisième module d'isolation primaire (370) et un troisième module d'isolation secondaire (380) ;
dans lequel le troisième module d'isolation primaire (370) est connecté entre le terminal de sortie du cinquième module de conversion de signal (352) et le terminal d'entrée du sixième module de conversion de signal (354), et le troisième module d'isolation secondaire (380) est connecté entre le terminal de sortie du sixième module de conversion de signal (354) et le dispositif de commande (160).

7. Circuit de surveillance en temps réel d'impulsions pour un instrument de thérapie d'une tumeur selon la revendication 1, dans lequel l'ordinateur obtient une impédance de charge selon le courant d'impulsion reçu et la tension d'impulsion reçue.

8. Circuit de surveillance en temps réel d'impulsions pour un instrument de thérapie d'une tumeur selon l'une quelconque des revendications 1 à 7, dans lequel le dispositif de commande (160) comprend une puce de traitement (462) et un circuit d'acquisition AN (464) connecté à la puce de traitement (462).

9. Instrument de thérapie d'une tumeur, comprenant un ordinateur (510), et le circuit de surveillance en temps réel d'impulsions (520) selon l'une quelconque des revendications 1 à 8 ;
dans lequel l'ordinateur (510) est connecté au dispositif de commande (160).

10. Instrument de thérapie d'une tumeur selon la revendication 9, comprenant en outre une alimentation électrique à haute tension ; dans lequel un terminal de sortie de l'alimentation électrique à haute tension est connectée au banc de condensateurs (110), et un terminal de commande de l'alimentation électrique à haute tension est connecté au dispositif de commande (160).
